# EUROPEAN PATENT SPECIFICATION

(11) **EP 2 208 735 B1**
(45) Date of publication and mention of the grant of the patent: **28.09.2011**
(21) Application number: 08846202.3
(22) Date of filing: 28.10.2008
(51) Int. Cl.: C07K 14/47, A23K 1/16, A23L 1/305, A61K 38/00, A61P 19/08, C07K 2/00

(54) **FOOD MATERIAL FOR PROMOTING THE DIFFERENTIATION OF OSTEOBLAST AND INHIBITING THE DIFFERENTIATION OF OSTEOCLAST**
NAHRUNGSMITTEL ZUR FÖRDERUNG DER DIFFERENZIERUNG VON OSTEOBLASTEN UND ZUR INHIBIERUNG DER DIFFERENZIERUNG VON OSTEOCLASTEN
MATIÈRE ALIMENTAIRE FAVORISANT LA DIFFÉRENCIATION D'UN OSTÉOBLASTE ET INHIBANT LA DIFFÉRENCIATION D'UN OSTÉOCLASTE

(30) Priority: 01.11.2007 JP 2007285394
(43) Date of publication of application: 21.07.2010
(73) Proprietor: Megmilk Snow Brand Co., Ltd., Sapporo-shi Hakkaido 0650043 (JP)
(72) Inventor: SERIZAWA, Atsushi, Kawagoe-shi Saitama 350-1165 (JP); MORITA, Yoshikazu, Kawagoe-shi Saitama 350-1165 (JP); UETSUJI, Daisuke, Kawagoe-shi Saitama 350-1165 (JP); ONO, Aiko, Kawagoe-shi Saitama 350-1165 (JP); MATSUYAMA, Hiroaki, Kawagoe-shi Saitama 350-1165 (JP); HIGURASHI, Satoshi, Kawagoe-shi Saitama 350-1165 (JP)
(74) Representative: Wakerley, Helen Rachael
(86) International application number: PCT/JP2008/003065
(87) International publication number: WO 2009/057281

(56) References cited:
- EP-A1- 1 656 948
- EP-A1- 1 669 084
- WO-A1-00/33077
- JP-A- 8 151 331
- JP-A- 10 007 585
- JP-A- 2001 346 519
- JP-A- 2004 115 509
- JP-A- 2004 115 509
- JP-A- 2005 060 321

## Description

### TECHNICAL FIELD

The present invention relates to a milk protein fraction or a milk protein fraction degradation product that exhibits an osteoblast differentiation promoting effect and an osteoclast differentiation inhibiting effect.
Since the milk protein fraction or the milk protein fraction degradation product according to the present invention exhibits an osteoblast differentiation promoting effect and an osteoclast differentiation inhibiting effect, the milk protein fraction or the milk protein fraction degradation product is useful as an osteoblast differentiation promoting and osteoclast differentiation inhibiting agent that aims at preventing or treating bone diseases or strengthening a bone, and is also useful as an active ingredient of a pharmaceutical, food and drink, or feed for preventing or treating bone diseases or strengthening a bone, or promoting the differentiation of osteoblast and inhibiting the differentiation of osteoclast.

### BACKGROUND ART

In recent years, various bone diseases, such as osteoporosis, bone fractures, lumbago or the like have increased along with the progressive increase in the elderly population. In a bone tissue, osteogenesis and bone resorption incessantly occur. In a young person, a balance between osteogenesis and bone resorption is kept, but the balance is disrupted tobone resorption owing to various causes with aging (uncoupling). Continuance of this state for a long period of time makes the bone tissue fragile, resulting in occurrence of various bone diseases, such as osteoporosis, bone fractures, and lumbago. It is considered that prevention of the uncoupling enables prevention of various bone diseases, such as osteoporosis, bone fractures, and lumbago.

Conventionally, in order to prevent the uncoupling to prevent or treat bone diseases, the following methods have been performed: (1) calcium supplementation by diet, (2) light exercise, (3) insolation, (4) medication, and the like. For calcium supplementation by diet, there are used calcium salts, such as calcium carbonate, calcium phosphate or the like, or natural calcium agents such as eggshell, fish bone powder or the like. However, these materials are not necessarily suitable for oral intake. Jogging, walking, or the like may be recommended as light exercise. However, even light exercise is troublesome for a person whose body has weakened, and it is almost impossible for a bedridden old person to do exercise. It is considered that insolation is a good means to supplement activated vitamin D₃, but it is not sufficient in itself. 1 α-Hydroxyvitamin D₃, a calcitonin preparation, or the like is used for administration of a pharmaceutical, and is known to be effective for treating osteoporosis. However, these substances are pharmaceuticals themselves and cannot be used as a food material.

The inventors of the present invention have searched for a bone-strengthening factor contained in milk in order to obtain a bone-strengthening substance that can be used as a food material. As a result, the inventors found that a protein and a peptide mixture obtained by removing a salt derived from a milk serum from a water-soluble fraction of a milk serum protein exhibit a bone-strengthening effect (see Patent Document 1, for example). The inventors found that a fraction obtained by subjecting an aqueous solution of the protein and the peptide mixture to an ethanol treatment, a heat treatment, a salting treatment, and an ultrafiltration membrane treatment exhibits an osteoblast growth promoting effect and a bone-strengthening effect (see Patent Documents 2 and 3, for example). The inventors further found that a basic protein contained in milk exhibits an osteoblast growth promoting effect, a bone-strengthening effect, and a bone resorption prevention effect (see Patent Document 4, for example).
Patent Document 1: Japanese Patent No. 3160862
Patent Document 2: Japanese Patent No. 3092874
Patent Document 3: JP-A-H05-320066
Patent Document 4: Japanese Patent No. 3112637

### DISCLOSURE OF THE INVENTION

### PROBLEMS TO BE SOLVED BY THE INVENTION

Based on the finding of a milk protein fraction or a milk protein fraction degradation product that exhibits an osteoblast differentiation promoting effect and an osteoclast differentiation inhibiting effect and can be used as a food material, an object of the present invention is to provide an osteoblast differentiation promoting and osteoclast differentiation inhibiting agent containing the milk protein fraction or the milk protein fraction degradation product that exhibits an osteoblast differentiation promoting effect and an osteoclast differentiation inhibiting effect, and a pharmaceutical, food, drink, or feed containing the milk protein fraction or the milk protein fraction degradation product that exhibits an osteoblast differentiation promoting effect and an osteoclast differentiation inhibiting effect.

### MEANS FOR SOLVING THE PROBLEMS

The inventors searched for a novel bone-strengthening material, and found that a fraction exhibiting a high osteoblast differentiation promoting effect and an osteoclast differentiation inhibiting effect as compared with a known food material could be obtained. Based on those findings, the inventors thus obtained an osteoblast differentiation promoting and osteoclast differentiation inhibiting agent containing the milk protein fraction or the milk protein fraction degradation product that exhibits an osteoblast differentiation promoting effect and an osteoclast differentiation inhibiting effect, and a pharmaceutical, food, drink, or feed containing the milk protein fraction or the milk protein fraction degradation product that exhibits an osteoblast differentiation promoting effect and an osteoclast differentiation inhibiting effect.

Specifically, the present invention is constituted as follows :
(A) A milk protein fraction characterized in that
   (1) the milk protein fraction is derived from milk,
   (2) the milk protein fraction contains proteins having a molecular weight of 75,000 to 80,000 daltons determined by sodium dodecyl sulfate-polyacrylamide gel electrophoresis (SDS-PAGE),
   (3) the milk protein fraction contains 13 to 15 wt% of basic amino acids in the constituent amino acid composition, and has a basic amino acid/acidic amino acid ratio of 0.5 to 0.7, and
   (4) the milk protein fraction has an osteoblast differentiation promoting effect and an osteoclast differentiation inhibiting effect.
(B) A milk protein fraction degradation product obtained by degrading the above milk protein fraction with a protease.
(C) An osteoblast differentiation promoting and osteoclast differentiation inhibiting agent comprising the milk protein fraction or the milk protein fraction degradation product according to (A) or (B), respectively.
(D) An osteoblast differentiation promoting and osteoclast differentiation inhibiting pharmaceutical comprising the milk protein fraction according to (A) or the milk protein fraction degradation product according to (A) or (B), respectively.
(E) An osteoblast differentiation promoting and osteoclast differentiation inhibiting food or drink comprising the milk protein fraction or the milk protein fraction degradation product according to (A) or (B), respectively.
(F) An osteoblast differentiation promoting and osteoclast differentiation inhibiting feed comprising the milk protein fraction or the milk protein fraction degradation product according to (A) or (B), respectively.

### EFFECTS OF THE INVENTION

The osteoblast differentiation promoting and osteoclast differentiation inhibiting agent containing the milk protein fraction or the milk protein fraction degradation product that exhibits an osteoblast differentiation promoting effect and an osteoclast differentiation inhibiting effect as an active ingredient, and the osteoblast differentiation promoting and osteoclast differentiation inhibiting pharmaceutical, food, drink, or feed containing the milk protein fraction or the milk protein fraction degradation product according to the present invention that exhibits an osteoblast differentiation promoting effect and an osteoclast differentiation inhibiting effect promotes the differentiation of the osteoblast and inhibits the differentiation and growth of the osteoclast in a body when taken orally.
Therefore, the agent containing the milk protein fraction or the milk protein fraction degradation product that exhibits an osteoblast differentiation promoting effect and an osteoclast differentiation inhibiting effect as an active ingredient, and the osteoblast differentiation promoting and osteoclast differentiation inhibiting pharmaceutical, food, drink, or feed containing the milk protein fraction or the milk protein fraction degradation product that exhibits an osteoblast differentiation promoting effect and an osteoclast differentiation inhibiting effect according to the present invention exhibit an osteoblast differentiation promoting effect and an osteoclast differentiation inhibiting effect by promoting the differentiation of the osteoblast and inhibits the differentiation of the osteoclast in the living body of a human or an animal, and are effective for suppressing a decrease in bone mass due to osteoporosis or the like.

### BRIEF DESCRIPTION OF THE DRAWINGS

Fig. 1 is a view showing an osteoclast differentiation and maturation inhibiting effect of a milk protein fraction (Test example 2).
Fig. 2 is a view showing a bone density increasing effect in mice which were administered a milk protein fraction (Test example 3).

### BEST MODE FOR CARRYING OUT THE INVENTION

The present invention relates to an osteoblast differentiation promoting agent including a milk protein fraction or a milk protein fraction degradation product that exhibits an osteoblast differentiation promoting effect and an osteoclast differentiation inhibiting effect, as well as a pharmaceutical, food or drink, and feed including a milk protein fraction or a milk protein fraction degradation product that exhibits an osteoblast differentiation promoting and osteoclast differentiation inhibiting effect.

The milk protein fraction according to the present invention may be obtained by bringing a milk raw material, such as skim milk, milk serum or the like into contact with a cation-exchange resin, washing the cation-exchange resin with 0.2M sodium chloride aqueous solution, and eluting the milk protein adsorbed on the cation-exchange resin using a 0.35M sodium chloride eluant. Note that salt such as a potassium salt, an ammonium salt, a phosphate, an acetate, a carbonate, or the like may be used in addition to sodium chloride. The milk protein fraction according to the present invention may be obtained by appropriately adjusting the ionic strength of the washing agent to 0.15 to 0.25 and the ionic strength of the elution solution to 0.3 to 0.4. Furthermore, the milk protein fraction according to the present invention may be obtained by collecting the eluted fraction, desalting and concentrating the fraction using a reverse osmosis (RO) membrane, electrodialysis (ED), or the like, and optionally drying the resulting product. Examples of the reverse osmosis (RO) membrane include Desal-3 (manufactured by Desalination), HR-95 (manufactured by Dow Danmark), NTR-729HF (manufactured by Nitto Denko Corporation), and the like. Examples of an electrodialysis (ED) system include electrodialysis systems manufactured by Yuasa-Ionics Inc. and Nippon Rensui Co., Ltd.

As a method of obtaining a trace protein fraction derived from milk, a method of obtaining a protein fraction by bringing milk or a raw material derived from milk into contact with a cation exchanger, and eluting the basic protein fraction that is adsorbed on the cation exchanger using an eluant that has a pH of more than 5 and an ionic strength of more than 0.5 (JP-A-H05-202098), a method of obtaining a protein fraction using an alginic acid gel (JP-A-S61-246198), a method of obtaining a protein fraction from a milk serum using porous inorganic particles (JP-A-H01-86839), a method of obtaining a protein fraction from milk using a sulfated ester compound (JP-A-S63-255300), and the like have been known. Protein fractions obtained by those methods may be used in the present invention.

The milk protein fraction thus collected may be normally powdered by freeze-drying or the like before use.

The milk protein fraction that exhibits an osteoblast differentiation promoting effect and an osteoclast differentiation inhibiting effect used in the present invention preferably contains 13 to 15wt% of basic amino acids in the constituent amino acid composition, and has a basic amino acid/acidic amino acid ratio of 0.5 to 0.7. The effect of the present invention may not be achieved if the content of basic amino acids or the basic amino acid/acidic amino acid ratio is outside the above range. The product according to the present invention is a protein mixture containing proteins having a molecular weight of 75,000 to 80,000 daltons, and the isoelectric point thereof is 7.5 to 8.5 as a main ingredient.

The milk protein fraction degradation product has the same amino acid composition as that of the milk protein fraction. For example, a milk protein fraction degradation product having an average molecular weight of 4000 or less may be obtained by treating a milk protein fraction obtained by the above method with a protease such as pepsin, trypsin, chymotrypsin or the like, and optionally treating the resulting product with a protease such as pancreatin or the like. The milk protein fraction degradation product is normally powdered by freeze-drying or the like before use.

As milk or a raw material derived from milk which can be used as source of the milk protein fraction that exhibits an osteoblast differentiation promoting effect and an osteoclast differentiation inhibiting effect, cow milk, human milk, goat milk, ewe milk or the like may be given. Such milks may be used as is, or recombined milk, skim milk, whey, or the like derived from such milks may be used.

The milk protein fraction or the milk protein fraction degradation product that exhibits an osteoblast differentiation promoting effect and an osteoclast differentiation inhibiting effect and is an active ingredient may be used as is when administering the osteoblast differentiation promoting and osteoclast differentiation inhibiting agent according to the present invention. Note that it is also possible to use after being formulated into a powdered pharmaceutical, granules, a tablet, a capsule, a drinkable preparation, or the like in accordance with a conventional method. Moreover, the milk protein fraction or the milk protein fraction degradation product, as is or after formulating a preparation thereof, may be added to a nutrient preparation, food and drink, or the like to achieve an osteoblast differentiation promoting effect and an osteoclast differentiation inhibiting effect. Since the milk protein fraction or the milk protein fraction degradation product according to the present invention is relatively stable against heat, the milk protein fraction or the milk protein fraction degradation product can be heat-sterilized under conventional conditions.

In the present invention, in order to achieve an osteoblast differentiation promoting effect and an osteoclast differentiation inhibiting effect the dosage or the like may be appropriately determined taking account of weight, sex, age, and the like. The milk protein fraction or the milk protein fraction degradation product may be adjusted the formulating amount thereof so that a normal adult takes the milk protein fraction or the milk protein fraction degradation of the present invention in an amount of 10 to 100 mg/day. That is, the milk protein fraction or the milk protein fraction degradation product according to the present invention is effective at a low dosage. In the present invention, the ingredient having an osteoblast differentiation promoting effect and an osteoclast differentiation inhibiting effect exerts the osteoblast differentiation promoting effect and the osteoclast differentiation inhibiting effect when orally administered an osteoblast differentiation promoting and osteoclast differentiation inhibiting agent or a pharmaceutical, food and drink, or feed formulated the osteoblast differentiation promoting and osteoclast differentiation inhibiting agent

The present invention is further described below by way of reference examples, examples, and test examples. Note that the following examples merely illustrate several aspects of the present invention, and should not be construed as limiting the present invention.

### Reference Example 1

A milk protein fraction exhibiting a bone-strengthening effect which was commercially available was prepared in accordance with the following method (see Japanese Patent No. 3112637).
A column (diameter: 10 cm) loaded with 0.5 litters of sulfonated Chitopearl (cation-exchange resin; manufactured by Fuji Spinning Co., Ltd.) was sufficiently washed with deionized water. After passing 501 of unsterilized skim milk through the column at a flow rate of 100 ml/min, the column was sufficiently washed with deionized water. 2.5 1 of a 0.05M phosphate buffer (pH 7.0) containing 0.95M sodium chloride was then passed through the column to elute proteins adsorbed on the resin. The eluate was desalted and concentrated by means of a reverse osmosis (RO) membrane treatment, and then freeze-dried to obtain a powdery milk protein fraction. The above procedure was repeated twice to obtain 10⁴ g of a protein fraction. The protein fraction had an isoelectric point of 7.0 to 8.5. The content of basic amino acids in the protein fraction was 17.8%.

### Example 1

A column (diameter: 10 cm) loaded with 0.5 1 of sulfonated Chitopearl (cation-exchange resin; manufactured by Fuji Spinning Co., Ltd.) was sufficiently washed with deionized water. After passing 501 of unsterilized skim milk through the column at a flow rate of 100 ml/min, the column was sufficiently washed with 0.05M phosphate buffer (pH 7.0) containing 0.15M sodium chloride. 2.51 of a 0.05M phosphate buffer (pH 7.0) containing 0.3M sodium chloride was then passed through the column to elute proteins adsorbed on the resin. The eluate was desalted and concentrated by means of a reverse osmosis (RO) membrane treatment, and then freeze-dried to obtain a powdery milk protein fraction. The above procedure was repeated three times to obtain 55.4 g of a protein fraction. The protein fraction comprised protein with a molecular weight of 75,000 to 80,000 daltons and the isoelectric point thereof was 7.5 to 8.5. The content of basic amino acids in the constituent amino acid contained in the protein fraction was 13 to 15%. The protein fraction had a basic amino acid/acidic amino acid ratio of 0.5 to 0.7.

### Example 2

A column (diameter: 10 cm) loaded with 0.5 1 of sulfonated Chitopearl (cation-exchange resin; manufactured by Fuji Spinning Co., Ltd.) was sufficiently washed with deionized water. After passing 50 1 of unsterilized skim milk through the column at a flow rate of 100 ml/min, the column was sufficiently washed with a 0.05M phosphate buffer (pH 7.0) containing 0.25M sodium chloride. 2.5 1 of a 0.05M phosphate buffer (pH 7.0) containing 0.4M sodium chloride was then passed through the column to elute proteins adsorbed on the resin. The eluate was desalted and concentrated by means of a reverse osmosis (RO) membrane treatment, and then freeze-dried to obtain a powdery milk protein fraction. The above procedure was repeated two times to obtain 37.3 g of a protein fraction. The protein fraction comprised protein with a molecular weight of 75,000 to 80,000 daltons and an isoelectric point thereof was 7.5 to 8.5. The content of basic amino acids in the constituent amino acid contained in the protein fraction was 13 to 15%. The protein fraction had a basic amino acid/acidic amino acid ratio of 0.5 to 0.7.

### Example 3

55.4 g of the milk protein fraction obtained in Example 1 was dissolved in 101 of distilled water. After adding pepsin (manufactured by Kanto Kagaku Co., Ltd.) so as to be the concentration of 2%, the milk protein fraction was hydrolyzed at 37°C for one hour with stirring. After the mixture was neutralized to pH 6.8 with a sodium hydroxide solution, 1% pancreatin (manufactured by Sigma) was added thereto. The mixture was then reacted at 37°C for two hours. After completion of the reaction, the protease was inactivated by heating the mixture at 80°C for 10 minutes to obtain 54.2 g of a milk protein fraction degradation product.

### Example 4

37.3 g of the milk protein fraction obtained in Example 2 was dissolved in 81 of distilled water. After adding trypsin (manufactured by Kanto Kagaku Co., Ltd.) so as to be the concentration of 2%, the milk protein fraction was hydrolyzed at 37°C for one hour with stirring. After the mixture was neutralized to pH 6.6 with a sodium hydroxide solution, 1% pancreatin (manufactured by Sigma) was added thereto. The mixture was then reacted at 37°C for two hours. After completion of the reaction, the protease was inactivated by heating the mixture at 80°C for 10 minutes to obtain 36.7 g of a milk protein fraction degradation product.

### Test example 1

The osteoblast differentiation promoting effect of the milk protein fraction obtained in Example 1 (Invention product) and the milk protein fraction obtained in Reference Example 1 (Reference example 1) was examined. Specifically, human preosteoblast MG63 cells were seeded to a 96-well plate at a cell density of 2 × 10⁴ cells/well, and cultured at 37°C for four days in a DMEM medium (manufactured by Flow Laboratories) containing a 10% fetal bovine serum in the presence of 5% CO₂ to obtain test cultured cells. After replacing the medium with a medium containing a 1 % fetal bovine serum, the milk protein fractions obtained in Example 1 and Reference Example 1 were added to the medium so that the final concentrations were 10 and 100 µg/ml, and the cells were cultured at 37°C for five days. The supernatant was collected, and the amount of type-I collagen in the supernatant was measured using a procollagen type-I C-peptide EIA kit (manufactured by Takara Bio Inc.) to determine osteoblast differentiation promoting activity. "Control" is a sample that the milk protein was not added. The amount of collagen is indicated by the ratio (%) of the amount of type-I collagen in each sample to the amount of type-I collagen in the Control. The results are shown in Table 1.

**Table 1**

| Sample | Final concentration | Osteoblast differentiation promoting activity |
|---|---|---|
| Control | -- | 100 ± 4.3 (± SD) |
| Milk protein fraction (Reference example 1) | 100 µg/ml | 157.2 ± 8.7 |
| Milk protein fraction (Example 1) | 100 µg/ml | 250.9 ± 12.7 |
| Milk protein fraction (Example 1) | 10 µg/ml | 180.3 ± 5.6 |

The amount of type-I collagen in any sample to which the milk protein fraction obtained in Example 1 or Reference example 1 was added was increased in comparison with that of the Control. This indicates that the milk protein fraction exhibited an osteoblast differentiation promoting effect. The milk protein fraction obtained in Example 1 exhibited a significant mass production ability in type-I collagen production compared with the milk protein fraction obtained in Reference example 1. This indicates that the milk protein fraction obtained in Example 1 exhibited a higher osteoblast differentiation promoting effect.

### Test example 2

ST2 cells were seeded to a 96-well plate so that the cell density was 2×10⁴ cells/well, and cultured in a 10% FBS-containing α-MEM medium (manufactured by GIBCO) in the presence of 5% CO₂ at 37°C for two days. Marrow cells collected from the thighbone of a ddy mouse (male, 7 or 8 weeks old) were seeded to the ST2 cell layer, and cultured at 37°C for 24 hours in the presence of 5% CO₂. The culture solution was removed, and a 1.0% FBS-containing α-MEM medium containing 1×10⁻⁸ M 1.25(OH₂)D₃ and 1×10⁻⁷ M dexamethasone was added to the plate (90 µl/well). Subsequently, the milk protein fraction obtained in Example 1 (Invention) or the milk protein fraction obtained in Reference example 1 (Reference example) was added to the plate (10 µl/well), and the cells were cultured at 37°C for three days in the presence of 5% CO₂. After replacing the medium, the cells were further cultured for three days. After completion of the culture, the culture solution was removed, and the resultant was washed with PBS, and then treated with an acetone-ethanol (1:1) solution for one minute and fixed. 1.5 mg/ml disodium p-nitrophenylphosphate-20 mM sodium tartrate-50 mM citrate buffer (pH: 4.5) were added (10 µl/well), and the mixture was reacted at room temperature for 30 minutes. The reaction was terminated by adding 1M sodium hydroxide solution (50 µl/well). The absorbance at 405 nm was then measured, and taken as an index of osteoclast differentiation and mutation.

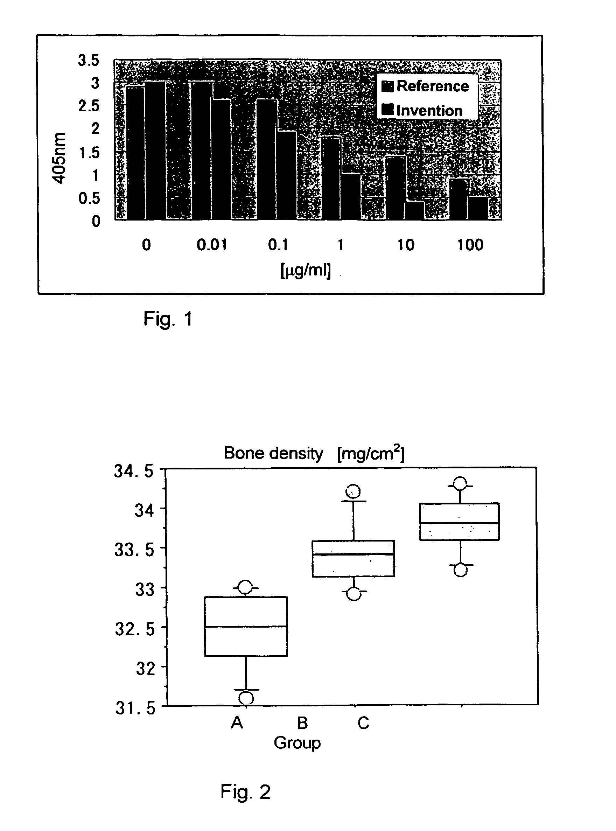

Fig. 1 shows the osteoclast differentiation and mutation test results. The higher the absorbance, the larger the osteoclast differentiation and mutation.
Therefore, it was found that the milk protein fraction obtained in Example 1 (Invention) suppressed osteoclast differentiation and mutation as compared with the milk protein fraction (Control) obtained in Reference example 1 (Reference example). Specifically, it was confirmed that the milk protein fraction according to the present invention significantly suppresses osteoclast differentiation and mutation as compared with the milk protein obtained in Reference example 1.

### Test Example 3

The protein fractions obtained in Reference example 1 and Example 1 were investigated about the bone-strengthening effect thereof by animal experiments. C57BL/6J female mice (4 weeks old) were used for the animal experiments. After preliminary feeding for one week, the ovary was exenterated from each rat. A calcium-deficient food was then fed to the rats for five weeks and the rats were provided to the animal experiments. The mice from which the ovary was exenterated and which were fed a calcium-deficient food had osteoporosis. The mice having osteoporosis were divided into three test groups of a Control group (Group A) that was not administered a milk protein fraction, a group (Group B) that was administered 0.1 wt% of the milk protein fraction obtained in Reference example 1, and a group (Group C) that was administered 0.1 wt% of the milk protein fraction obtained in Example 1 (six mice/each group). A test feed shown in Table 2 was fed to each group for four months. The nitrogen content in each test feed was equally adjusted to 17.06% using casein. Each test feed was blended 300 mg of calcium, 230 mg of phosphorus, and 50 mg of magnesium per 100 g of the test feed.

**Table 2**

| | | Group | |
|---|---|---|---|
| | A | B | C |
| Casein | 20.0 | 19.9 | 19.9 |
| Cornstarch | 15.0 | 15.0 | 15.0 |
| Cellulose | 5.0 | 5.0 | 5.0 |
| Corn oil | 5.0 | 5.0 | 5.0 |
| Vitamin mix | 1.0 | 1.0 | 1.0 |
| Mineral mix | 2.65 | 2.65 | 2.65 |
| Sucrose | 51.05 | 51.05 | 51.05 |
| DL-Methionine | 0.3 | 0.3 | 0.3 |
| Milk protein fraction obtained in Reference example 1 | - | 0.1 | - |
| Milk protein fraction obtained in Example 1 | - | - | 0.1 |

| | | | |
|---|---|---|---|
| (wt%) | | | |

The thighbone was exenterated from each mouse that was fed for four months, and the bone density was measured in accordance with dual energy X-ray absorptiometry (DEXA) method using a bone mineral analyzer ("DCS-600" manufactured by Aloka Co., Ltd.). The thighbone density of the Control group was also measured. The results are shown in Fig. 2. As shown in Fig. 2, after feeding the test feed for four months, the thighbone densities of the mice of the group (Group B) that was administered the milk protein fraction obtained in Reference example 1 and the group (Group C) that was administered the milk protein fraction obtained in Example 1 were significantly higher than those of the mice of the Control group. Each milk protein fraction showed a bone density increase effect (i.e., osteoblast differentiation promoting effect and osteoclast differentiation inhibiting effect). However, the effect achieved by the milk protein fraction of the product of the present invention was higher than that of the milk protein fraction obtained in Reference example 1.
In addition, similar effects were observed in the cases using the milk protein fractions degradation products obtained in Examples 3 and 4 (not shown in Fig. 2).

### Example 5

100 mg of the milk protein fraction obtained in Example I was added with 93.4 g of hydrated crystalline glucose, 5 g of calcium carbonate, 1 g of a sugar ester, and 0.5 g of flavor, and the mixture was mixed. The resultant was then formed into a tablet to obtain an osteoblast differentiation promoting and osteoclast differentiation inhibiting agent according to the present invention.

### Example 6

The components were mixed in accordance with the composition shown in Table 3 to obtain a dough. The dough was formed and baked to produce a cookie for promoting the osteoblast differentiation and inhibiting the osteoclast differentiation.

**Table 3**

| | |
|---|---|
| Flour | 50.0 (wt%) |
| Sugar | 20.0 |
| Salt | 0.5 |
| Margarine | 12.5 |
| Egg | 12.1 |
| Water | 4.0 |
| Sodium hydrogencarbonate | 0.1 |
| Ammonium bicarbonate | 0.2 |
| Calcium carbonate | 0.5 |
| Milk protein fraction powder (Example 1) | 0.1 |

### Example 7

A fruit juice drink for promoting the osteoblast differentiation and inhibiting the osteoclast differentiation having a composition shown in Table 4 was produced.

**Table 4**

| | |
|---|---|
| Isomerized sugar mix | 15.0 (wt%) |
| Fruit juice | 10.0 |
| Citric acid | 0.5 |
| Milk protein fraction powder (Example 1) | 0.5 |
| Flavor | 0.1 |
| Calcium | 0.1 |
| Water | 73.8 |

### Example 8

The ingredients were mixed in accordance with the formulation shown in Table 5 to produce a dog food for promoting the osteoblast differentiation and inhibiting the osteoclast differentiation.

**Table 5**

| | |
|---|---|
| Milk protein fraction powder (Example 1) | 2.5 (wt%) |
| Powdered skim milk | 13.5 |
| Soybean cake | 12.0 |
| Soybean oil | 4.0 |
| Corn oil | 2.0 |
| Palm oil | 27.0 |
| Corn starch | 14.0 |
| Wheat powder | 9.0 |
| Wheat bran | 2.0 |
| Vitamin mix | 9.0 |
| Mineral mix | 2.0 |
| Cellulose | 3.0 |

### Example 9

Each ingredient was mixed in accordance with the formulation shown in Table 6, and formed under pressure to produce an osteoblast differentiation promoting and osteoclast differentiation inhibiting tablet containing the milk protein fraction degradation product obtained in Example 3.

**Table 6**

| | |
|---|---|
| Hydrous crystalline glucose | 59.4 (wt%) |
| Milk protein fraction degradation product (Example 3) | 16.0 |
| Corn starch | 12.0 |
| Cellulose | 4.0 |
| Corn oil | 4.0 |
| Vitamin mix (including choline) | 1.0 |
| Mineral mix | 3.6 |

### Example 10

Each ingredient was mixed in accordance with the formulation shown in Table 7, and emulsified at 85°C to produce a processed cheese for promoting the osteoblast differentiation and inhibiting the osteoclast differentiation containing the milk protein fraction degradation product obtained in Example 4.

**Table 7**

| | |
|---|---|
| Gouda cheese | 43.0 (wt%) |
| Cheddar cheese | 43.0 |
| Sodium citrate | 2.0 |
| Milk protein fraction degradation product (Example 4) | 0.5 |
| Calcium derived from milk | 1.0 |
| Water | 10.5 |

### INDUSTRIAL APPLICABILITY

The milk protein fraction or the milk protein fraction degradation product according to the present invention inhibits osteoclast differentiation, and therefore exhibits a bone-strengthening effect. Moreover, since the milk protein fraction or the milk protein fraction degradation product according to the present invention is effective for suppressing a decrease in bone mass, such as osteoporosis, the milk protein fraction or the milk protein fraction degradation product according to the present invention may be used for an osteoblast differentiation promoting and osteoclast differentiation inhibiting agent containing the milk protein fraction or the milk protein fraction degradation product as an active ingredient, and an osteoblast differentiation promoting and osteoclast differentiation inhibiting pharmaceutical, food, drink, or feed containing the milk protein fraction or the milk protein fraction degradation product that exhibits an osteoblast differentiation promoting effect and an osteoclast differentiation inhibiting effect.

## Claims

1. A milk protein fraction having following characteristics (1) to (4); (1) the milk protein fraction is derived from milk, (2) the milk protein fraction contains proteins having a molecular weight of 75,000 to 80,000 daltons determined by sodium dodecyl sulfate-polyacrylamide gel electrophoresis (SDS-PAGE), (3) the milk protein fraction contains 13 to 15 wt% of basic amino acids in the constituent amino acid composition, and has a basic amino acid/acidic amino acid ratio of 0.5 to 0.7, and (4) the milk protein fraction has an osteoblast differentiation promoting effect and an osteoclast differentiation inhibiting effect.

2. A milk protein fraction degradation product obtained by degrading the milk protein fraction according to claim 1 with a protease.

3. An osteoblast differentiation promoting and osteoclast differentiation inhibiting agent comprising the milk protein fraction according to claim 1 or the milk protein fraction degradation product according to claim 2.

4. An osteoblast differentiation promoting and osteoclast differentiation inhibiting pharmaceutical comprising the milk protein fraction according to claim 1 or the milk protein fraction degradation product according to claim 2.

5. An osteoblast differentiation promoting and osteoclast differentiation inhibiting food or drink comprising the milk protein fraction according to claim 1 or the milk protein fraction degradation product according to claim 2.

6. An osteoblast differentiation promoting and osteoclast differentiation inhibiting feed comprising the milk protein fraction according to claim 1 or the milk protein fraction degradation product according to claim 2.

## Patentansprüche

1. Milchprotelnfraktion mit den folgenden Kennzeichen (1) bis (4); (1) die Milchproteinfraktion wird aus Milch gewonnen, (2) die Milchproteinfraktion enthält Proteine mit einem Molekulargewicht von 75.000 bis 80.000 Dalton, bestimmt anhand von Natriumdodecylsulfat-Polyacrylamid-Gelelektrophorese (SDS-PAGE), (3) die Milchproteinfraktion enthält in der konstituierenden Aminosäurezusammensetzung 13 bis 15 Gew.-% an basischen Aminosäuren und weist ein Verhältnis von basischen Aminosäuren/sauren Aminosäuren von 0,5 bis 0,7 auf, und (4) die Milchproteinfraktion besitzt eine die Osteoblastendifferenzierung fördernde sowie eine die Osteoclastendifferenzierung hemmende Wirkung.

2. Milchproteinfraktion-Abbauprodukt, erhalten durch Abbau der Milchproteinfraktion nach Anspruch 1 mithilfe einer Protease.

3. Osteoblastendifferenzierung fördernde sowie Osteoclastendifferenzierung hemmende Substanz, umfassend die Milchproteinfraktion nach Anspruch 1 oder das Milchproteinfraktion-Abbauprodukt nach Anspruch 2.

4. Osteoblastendifferenzierung förderndes sowie Osteoclastendifferenzierung hemmendes Arzneimittel, umfassend die Milchproteinfraktion nach Anspruch 1 oder das Milchproteinfraktion-Abbauprodukt nach Anspruch 2.

5. Osteoblasteridiffererizierung förderndes sowie Osteoclastendifferenzierung hemmendes Nahrungsmittel oder Getränk, umfassend die Milchproteinfraktion nach Anspruch 1 oder das Milchproteinfraktion-Abbauprodukt nach Anspruch 2.

6. Osteoblastendifferenzierung förderndes sowie Osteoclasteridifferenzierung hemmendes Futtermittel, umfassend die Milchproteinfraktion nach Anspruch 1 oder das Milchproteinfraktion-Abbauprodukt nach Anspruch 2.

## Revendications

1. Fraction de protéines lactiques présentant les caractéristiques (1) à (4) suivantes ; (1) la fraction de protéines lactiques est dérivée du lait, (2) la fraction de protéines lactiques contient des protéines ayant un poids moléculaire compris entre 75.000 et 80.000 daltons, déterminé par électrophorèse sur gel de polyacrylamide en présence de dodécylsulfate de sodium (SDS-PAGE), (3) la fraction de protéines lactiques contient 13 à 15 % en poids d'acides aminés basiques dans la composition d'acides aminés constituants, et a un rapport acides aminés basiques / acides aminés acides compris entre 0,5 et 0,7, et (4) la fraction de protéines lactiques exerce un effet favorisant la différenciation d'un ostéoblaste et un effet inhibant la différenciation d'un ostéoclaste.

2. Produit de dégradation de la fraction de protéines lactiques obtenu en dégradant la fraction de protéines lactiques selon la revendication 1 avec une protéase.

3. Agent favorisant la différenciation d'un ostéoblaste et inhibant la différenciation d'un ostéoclaste comprenant la fraction de protéines lactiques selon la revendication 1 ou le produit de dégradation de la fraction de protéines lactiques selon la revendication 2.

4. Produit pharmaceutique favorisant la différenciation d'un ostéoblaste et inhibant la différenciation d'un ostéoclaste comprenant la fraction de protéines lactiques selon la revendication 1 ou le produit de dégradation de la fraction de protéines lactiques selon la revendication 2.

5. Aliment ou boisson favorisant la différenciation d'un ostéoblaste et inhibant la différenciation d'un ostéoclaste comprenant la fraction de protéines lactiques selon la revendication 1 ou le produit de dégradation de la fraction de protéines lactiques selon la revendication 2.

6. Matière alimentaire favorisant la différenciation d'un ostéoblaste et inhibant la différenciation d'un ostéoclaste comprenant la fraction de protéines lactiques selon la revendication 1 ou le produit de dégradation de la fraction de protéines lactiques selon la revendication 2.
